# EUROPEAN PATENT APPLICATION

(11) **EP 3 578 641 A1**
(43) Date of publication of application: **11.12.2019**
(21) Application number: 18747960.5
(22) Date of filing: 01.02.2018
(51) Int. Cl.: C12N 5/0775, A61K 35/28, A61L 27/38, A61P 43/00

(54) **METHOD FOR PREPARING DENTAL PULP STEM CELLS FROM CELLS DERIVED FROM DENTAL PULP TISSUE**

(30) Priority: 03.02.2017 JP 2017019019
(71) Applicant: Kyushu University National University Corporation, Fukuoka-shi, Fukuoka 819-0395 (JP); Advanced Cell Technology and Engineering Ltd., Chuo-ku Tokyo 1040033 (JP)
(72) Inventor: YAMAZA Takayoshi, Fukuoka-shi Fukuoka 819-0395 (JP); SONODA Soichiro, Fukuoka-shi Fukuoka 819-0395 (JP); HAMAZONO Toshiro, Tokyo 104-0033 (JP)
(74) Representative: SSM Sandmair
(86) International application number: PCT/JP2018/003498
(87) International publication number: WO 2018/143378

(57) **Abstract**

[Problem] The present invention addresses the problem of providing a method for efficiently preparing dental pulp stem cells from dental pulp cells collected from dental pulp tissue. [Solution Means] The method for preparing dental pulp stem cells from dental pulp cells collected from dental pulp tissue according to the present invention includes: (a) a step for preculturing dental pulp cells using a culture medium that includes serum at 7-13% (v/v), the dental pulp cells being bonded to a culture dish; (b) a step for culturing the dental pulp cells bonded to the culture dish by step (a) at least for one day using a culture medium that includes serum at 7-13% (v/v); and (c) a step for culturing the dental pulp cells in a low-serum condition after step (b).

## Description

### TECHNICAL FIELD

The present invention relates to a method for preparing dental pulp stem cells from cells derived from dental pulp tissue.

### BACKGROUND ART

Mesenchymal stem cells, one of the somatic stem cells, is a collective term for stem cells, is considered to have differentiation potential into mesenchymal-lineage cells such as osteoblasts, adipocytes, muscle cells, and chondrocytes, and are known to be present in all mesenchymal tissues; therefore, the application in regenerative medicine to reconstruct bones, muscles, blood vessels, and nerves is expected.

Dental pulp stem cells derived from dental pulp tissue have been known as one of stem cells categorized into mesenchymal stem cells. Because dental pulp stem cells, especially dental pulp stem cells derived from deciduous teeth, show therapeutic effects in various disease models (Non-Patent Literatures 1-5), dental pulp stem cells have attracted attention as a source of stem cells used in regenerative medicine.

Here, stem cells are cytologically defined as cells that have the basic properties of self-renewal and pluripotency or multipotency. The basic properties include a stem cell property (sternness), which refer to the ability of stem cells to maintain pluripotency or multipotency, and cell division ability without becoming differentiated cells, are completely distinguishable from somatic cells. It is known that stem cells with low sternness have reduced ability to divide and multipotency (Non-Patent Literature 6).

When considering stem cell therapy in a clinic, therefore, the degree of the stem cell properties including sternness retained in isolated stem cells is considered to be a very important factor that determines their therapeutic effects (Non-Patent Literature 7).

Regarding isolation methods of mesenchymal stem cells including dental pulp stem cells, more favorable isolation conditions have been examined. For example, it is reported that stem cells with pluripotency were successfully isolated from mesenchymal tissue under a stress condition by enzyme treatment with dispase (Non-Patent Literature 8). Furthermore, it is reported in Non-Patent Literatures 9-11 that the stem cell properties are enhanced under hypoxic conditions.

In addition, Non-Patent Literature 1 discloses a method for obtaining SSEA-3- and CD105-positive pluripotent stem cells under external stress such as enzyme treatment, hypoxic condition, and low serum condition to mesenchymal stem cells or cultured mesenchymal cells.

### CITATION LIST

### PATENT LITERATURES

Patent Literature 1: Japanese Patent Application Publication No. 2016-28614

### NON-PATENT LITERATURES

Non-Patent Literature 1: Seo et al., "SHED repair critical-size calvarial defects in mice." Oral Dis. (2008)14:428-434.
Non-Patent Literature 2: Yamaza et al., "Immunomodulatory properties of stem cells from human exfoliated deciduous teeth." Stem Cell Res Ther. (2010)1:5.
Non-Patent Literature 3: Yamaza et al., "In vivo hepatogenic capacity and therapeutic potential of stem cells from human exfoliated deciduous teeth in liver fibrosis in mice." Stem Cell Res Ther. (2015)6:171.
Non-Patent Literature 4: Ma et al., "Cryopreserved dental pulp tissues of exfoliated deciduous teeth is a feasible stem cell resource for regenerative medicine." PLoS One. (2012)
Non-Patent Literature 5: Ma et al., "Transplantation of mesenchymal stem cells ameliorates secondary osteoporosis through interleukin-17-impaired functions of recipient bone marrow mesenchymal stem cells in MRL/lpr mice." Stem Cell Res Ther. (2015) 6:104.
Non-Patent Literature 6: Bose et al., "Aging induced loss of sternness with concomitant gain of myogenic properties of a pure population of CD34(+)/CD45(-) muscle derived stem cells." IntJ Biochem Cell Biol. (2016) 70:1-12.
Non-Patent Literature 7: Tobita et al., "Adipose tissue-derived mesenchymal stem cells and platelet-rich plasma: stem cell transplantation methods that enhance sternness." Stem Cell Res Ther. (2015) Nov 5;6:215.
Non-Patent Literature 8: Wakao et al., Multilineage-differentiating stress-enduring (Muse) cells are a primary source of induced pluripotent stem cells in human fibroblasts." Proc Natl Acad Sci U S A. (2011) 108:9875-9880.
Non-Patent Literature 9: D'Ippolito et al., "Schiller PC. Low oxygen tension inhibits osteogenic differentiation and enhances sternness of human MIAMI cells" Bone. (2006) 39:513-22.
Non-Patent Literature 10: Hung et al., "Hypoxia promotes proliferation and osteogenic differentiation potentials of human mesenchymal stem cells." J Orthop Res. (2012) 30:260-266.
Non-Patent Literature 11: Berniakovich and Giorgio, "Low oxygen tension maintains multipotency, whereas normoxia increases differentiation of mouse bone marrow stromal cells." Int J MolSci. (2013) 14:2119-2134.

### SUMMARY OF THE INVENTION

### PROBLEMS TO BE SOLVED BY THE INVENTION

The present invention provides a method for preparing dental pulp stem cells more efficiently from dental pulp tissue and a method in which stem cell properties of the obtained dental pulp stem cells are enhanced.

### MEANS FOR SOLVING THE PROBLEM

Recently, CD34 positive dental pulp stem cells have been identified (Pisciotta et al., 2015). According to this literature, it is reported that, compared to CD34 negative dental pulp stem cells, CD34 positive dental pulp stem cells exhibit a low cell proliferation rate and long maintenance of stem cell properties.

In addition, human bone marrow mesenchymal stem cells and dental pulp stem cells have been reported to express CD146 (Shi and Gronthos, 2003). CD146 (also known as MUC18, MCAM) is a 113 kDa cell adhesion molecule identified in human melanoma cells (Lehmann et al., 1987), and it has been shown that deciduous tooth stem cells also expressed CD146 on the cell surface (Non-Patent Literature 2). CD146 positive human bone marrow mesenchymal stem cells and dental pulp stem cells are frequently observed around blood vessels, suggesting an association with stem cell niche formation (Shi and Gronthos, 2003). In addition, it can be also considered that CD146 is an primitive marker of mesenchymal stem cells, because it was reported that decreased expression of CD146 promoted cellular senescence (Jin et al., 2016).

Furthermore, SSEA-4 (stage specific antigen-4) is known as a marker for pluripotent stem cells whose expression has been clarified in bone marrow mesenchymal stem cells (Gang et al., 2007). The expression of SSEA-4 has been reported on the cell surface of deciduous tooth stem cells (Yamaza et al., 2010). SSEA-4 is a cell surface molecule identified from teratocarcinoma cells (Kannagi et al., 1983), and is known as an primitive marker for pluripotent stem cells including ES cells and iPS cells (Henderson et al., 2002; Takahashi et al., 2007). Therefore, subpopulations of mesenchymal stem cells expressing SSEA-4 are considered to be very primitive populations.

For instance, if dental pulp stem cells, in which the expression of molecules listed above is enhanced, can be obtained, dental pulp stem cells having superior stem cell properties can be provided.

In addition, Non-Patent Literature 1 describes a method for obtaining the SSEA-3- and CD105-positive pluripotent stem cells as mentioned above, but it mentions that the obtained pluripotent stem cells were CD146-negative cells.

The present inventors have so far conducted isolation of deciduous tooth stem cells (Yamaza et al. , 2010) based on a colony forming unit-fibroblasts (CFU-F) colony formation method (Friedenstein et al., 1976). For example, in Non-Patent Literature 1, it is reported that, when dental pulp stem cells were collected from the dental pulp tissue of human deciduous teeth, the dental pulp stem cells were isolated by the CFU-F colony formation method using a 15% fetal bovine serum (FBS)-containing culture medium. As described above, Non-Patent Literature 8 and Non-Patent Literature 9 show that enhancement of stem cell properties was observed by applying stress to cells. We focused on the possibility to isolate stem cell populations expressing enhanced stem cell properties when stress is given by a method other than enzyme treatment or hypoxic condition. For example, conditional changes in adding timing and concentration of serum containing of growth factor to the culture medium might give stress to isolate stem cells with enhanced sternness. Meanwhile, no study has improved the influence of the timing of the serum addition in the culture medium upon the isolation process of dental pulp stem cells on the stem cell properties of dental pulp stem cells until now. Therefore, in order to solve the above problems, the present inventors focused attention on the influence of the serum concentration in the culture medium on dental pulp stem cells in the isolation protocol as described in Non-Patent Literature 2, and carefully studied the impact of the serum during the culture period.

As a result, surprisingly, we succeeded in obtaining dental pulp stem cells having more maintained stem cell properties by attaching a cell population containing dental pulp stem cells collected from the dental pulp tissue onto a culture dish, adding serum to the culture medium during a specific culture period for culture to form colonies containing dental pulp stem cells, and culturing the cells under a low serum or serum-free culture condition during the subsequent culture period. The present invention has been completed based on these findings.

That is, the present invention is as described below:
The present invention, in one aspect, includes
[1] a method for preparing dental pulp stem cells from dental pulp cells collected from dental pulp tissue, including the steps of:
   (a) preculturing the dental pulp cells using a culture medium containing serum at 7 to 13% (V/V), and attaching the dental pulp cells on a culture dish;
   (b) culturing the dental pulp cells, which are attached on the culture dish in the step (a), at least for one day using a culture medium containing serum at 7 to 13% (V/V) or a serum-free culture medium for stem cells; and
   (c) culturing the dental pulp cells in a low serum condition or a serum-free condition after the step (b).
   Here, in one embodiment, the method for preparing dental pulp stem cells from dental pulp cells collected from dental pulp tissue according to the present invention is,
[2] the method according to [1] above, wherein the step (c) is characterized in that the dental pulp cells are cultured both in a low serum condition or a serum-free condition and in a low nutritional condition.
   Furthermore, in one embodiment, the method for preparing dental pulp stem cells from dental pulp cells collected from dental pulp tissue of the present invention is,
[3] the method according to [1] or [2] above, and the method is characterized by including the step of seeding the dental pulp cells on a culture dish before the step (a) so as not to adhere to other cells, thereby forming cell colonies derived from single cells.
   Furthermore, in one embodiment, the method for preparing dental pulp stem cells from dental pulp cells collected from dental pulp tissue of the present invention is,
[4] The method according to any one of the above [1] to [3], wherein the serum used in the steps (a) and (b) is FBS.
   Furthermore, in one embodiment, the method for preparing dental pulp stem cells from dental pulp cells collected from dental pulp tissue of the present invention is,
[5] the method according to any one of the above [1] to [4], wherein the culture medium used in the step (a) and/or (b) is a culture medium containing serum at 10 to 13% (V/V), and cell colony containing dental pulp stem cells with enhanced expression of SSEA-4.
   Furthermore, in one embodiment, the method for preparing dental pulp stem cells from dental pulp cells collected from dental pulp tissue of the present invention is,
[6] the method according to any one of the above [1] to [5], wherein the method further includes the step of preparing the dental pulp cells by enzyme treatment of the dental pulp tissue before the step (a).
   Furthermore, in one embodiment, the method for preparing dental pulp stem cells from dental pulp cells collected from dental pulp tissue of the present invention is,
[7] the method according to any one of the above [1] to [6], wherein the method further includes (a') a washing step for replacing the culture medium after the step (a) and before the step (b).
   Furthermore, in one embodiment, the method for preparing dental pulp stem cells from dental pulp cells collected from dental pulp tissue of the present invention is,
[8] The method according to any one of the above [1] to [7], wherein the step (c) is a step of culturing dental pulp cells using the serum-containing culture medium containing or the serum-free culture medium for stem cells used in the step (b) without replacing the medium with a fresh culture medium.
   Furthermore, in one embodiment, the method for preparing dental pulp stem cells from dental pulp cells collected from dental pulp tissue of the present invention is,
[9] the method according to [8] above, wherein the total culture time of the steps (b) and (c) is at least 168 hours.
   Furthermore, in one embodiment, the method for preparing dental pulp stem cells from dental pulp cells collected from dental pulp tissue of the present invention is,
[10] the method according to any one of the above [1] to [9], wherein the dental pulp cells seeded on the culture dish in the step (a) are contained in the culture medium at a concentration of 0.01 × 10⁶ to 0.1 × 10⁶ cells/mL.
   Furthermore, in one embodiment, the method for preparing dental pulp stem cells from dental pulp cells collected from dental pulp tissue of the present invention is,
[11] the method according to any one of the above [1] to [10], wherein the preculture time of the step (a) is 24 to 48 hours.
   Moreover, another aspect of the present invention relates to,
[12] a method for producing a therapeutic implant material, including the step of producing a therapeutic implant using dental pulp stem cells obtained by the method according to any one of the above [1] to [11].
   Moreover, another aspect of the present invention relates to,
[13] dental pulp stem cells obtained by the method according to any one of the above [1] to [11], which are derived from single cell colony-derived dental pulp stem cells in which expression of SSEA-4 is enhanced.
   Here, the dental pulp stem cells of the present invention are,
[14] the dental pulp stem cells according to [13] above, wherein the cell colonies further have enhanced expression of CD146 or CD34 in addition to SSEA-4.
   Moreover, another aspect of the present invention relates to,
[15] a therapeutic implant material including the dental pulp stem cells according to [13] or [14] above.

### EFFECTS OF THE INVENTION

According to the method of the present invention, it is possible to efficiently prepare dental pulp stem cells from dental pulp cells by a simple method, eliminating the need for complicated operations such as enzyme treatment and hypoxic condition. In addition, dental pulp stem cells can be obtained at serum concentrations lower than conventionally used serum concentrations; therefore, the cost can be reduced, and it is preferable in particular when the manufacuture of a large amount of cells is required such as cell banks.

Furthermore, the method of the present invention makes it possible to prepare dental pulp stem cells with enhanced stem cell properties in addition to the efficient preparation of dental pulp stem cells.

### BRIEF DESCRIPTION OF THE DRAWINGS

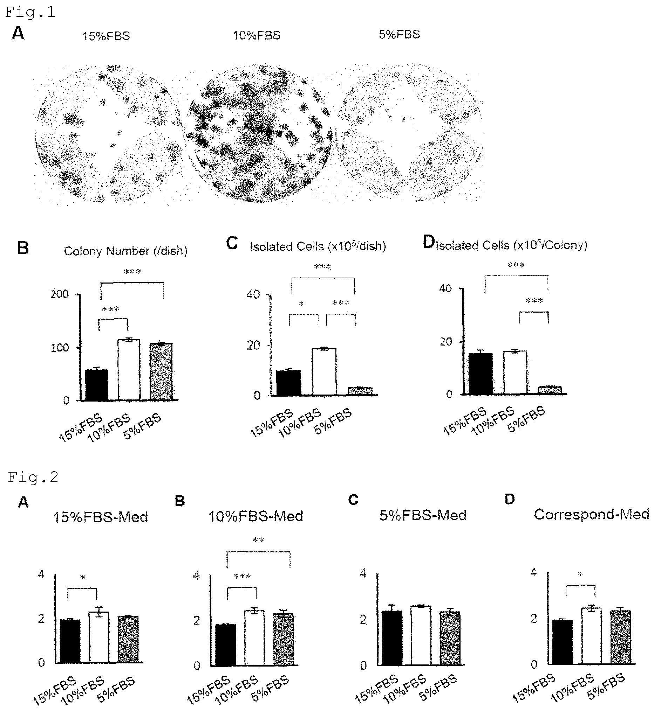

[Fig. 1] Fig. 1 shows the analysis results of the colony forming ability in Examples 2 and 3 below. Nucleated cells isolated from the dental pulp of deciduous teeth were seeded at 0.1 × 10⁶ cells. Fig. 1A shows images of the CFU-F colonies treated with toluidine blue staining. Also, Fig. 1B shows the number of the formed CFU-F colonies, Fig. 1C shows the number of the CFU-F-forming cells per culture dish, and Fig. 1D shows the number of cells per CFU-F colony. n = 3. Bar = average value ± SEM (%). *: P < 0.05, ***: P < 0.005.
[Fig. 2] Fig. 2 shows the analysis results of the cell proliferation ability in Example 4 below. Specifically, it shows the analysis results of the cell proliferation ability of deciduous tooth stem cells by a Brd-U uptake assay. Fig. 2A is a graph showing the results of the formation of adherent colonies under a culture medium containing 15% FBS after pre-incubation at each FBS concentration. Fig. 2B is a graph showing the results of the formation of adherent colonies under a culture medium containing 10% FBS after pre-incubation at each FBS concentration. Fig. 2C is a graph showing the results of the formation of adherent colonies under a culture medium containing 5% FBS after pre-incubation at each FBS concentration. Fig. 2D is a graph showing a comparison of the results of cultures at common FBS concentrations throughout the culture. n = 3. Bar = average value ± SEM (%). *: P < 0.05, **: P < 0.05, ***: P < 0.005.

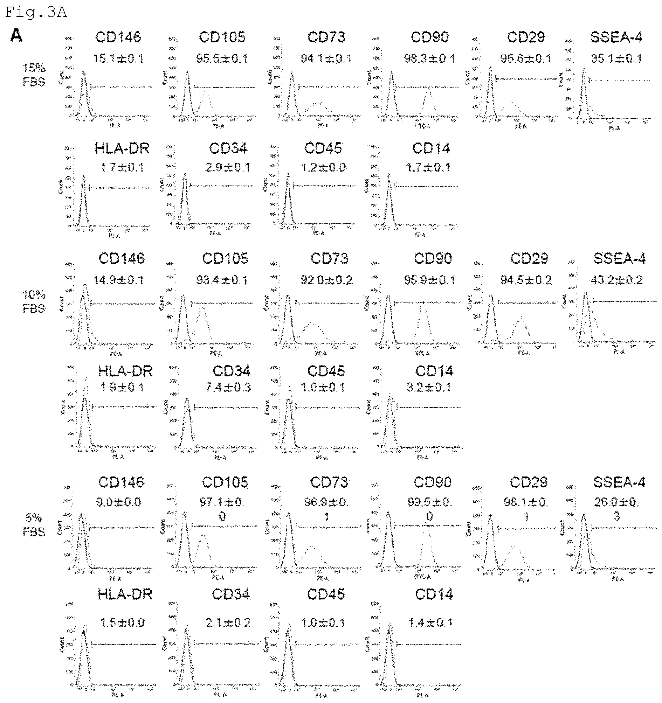

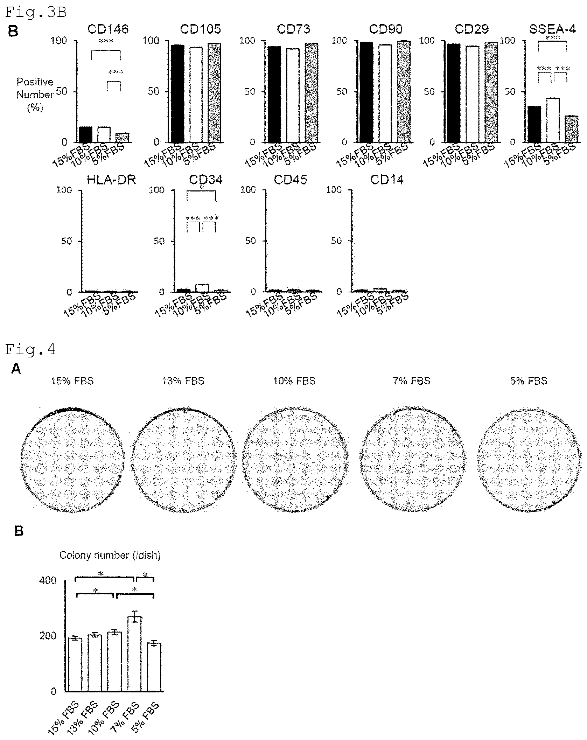

[Fig. 3A] Fig. 3 shows graphs showing the analysis results of the expression of cell surface antigens by flow cytometric assay in Example 5 below. Fig. 3A is histograms after the flow cytometric analysis. In Fig. 3A, the black lines represent the groups stained with specific anti-cell surface antigen antibodies, and the gray lines represent the groups stained with the corresponding isotype-matched antibodies. Numbers indicate the mean value ±SEM (%) of the positive rate. Fig. 3B is graphs showing the positive rate of each surface antigen. n = 3. Bar = average value ± SEM (%). *: P < 0.05, ***: P < 0.005.
[Fig. 4] Fig. 4 shows the analysis results of colony forming ability in Example 7 below. Nucleated cells isolated from the dental pulp tissue of deciduous teeth were seeded at 0.1 × 10⁶ cells. Fig. 4A shows images obtained by toluidine blue staining of CFU-F colonies obtained after culture. Also, Fig. 4B shows the number of CFU-F colony forming cells. n = 3. 15% FBS: an isolation group using 15% FBS, 13% FBS: an isolation group using 13% FBS, 10% FBS: an isolation group using 10% FBS, 7% FBS: an isolation group using 7% FBS, 5% FBS: an isolation group using 5% FBS. Bar = average value ± SEM (%). *: P < 0.05.

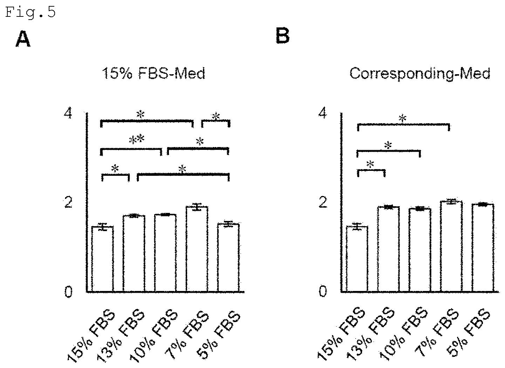

[Fig. 5] Fig. 5 shows the analysis results of the cell proliferation ability in Example 8 below. Specifically, it shows the analysis results of cell proliferation of deciduous tooth stem cells by Brd-U uptake assay. Fig. 5A shows the analysis results using 15% FBS-containing culture medium (15% FBS-Med) after preculturing at the indicated FBS concentration. Fig. 5B shows the analysis results using a medium (Correspond-Med) with the same FBS concentration in both the preculture and growth culture. n = 3. 15% FBS: an isolation group using 15% FBS, 13% FBS: an isolation group using 13% FBS, 10% FBS: an isolation group using 10% FBS, 7% FBS: an isolation group using 7% FBS, 5% FBS: an isolation group using 5% FBS. Bar = average value ± SEM (%). *: P < 0.05, ***: P < 0.005.

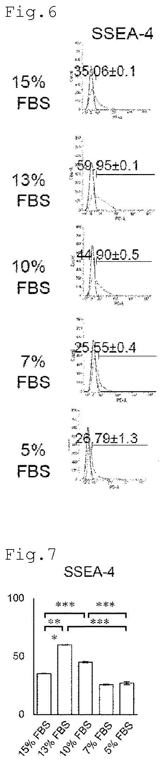

[Fig. 6] Fig. 6 is graphs showing the analysis results of the expression of SSEA-4 by flow cytometric assay in Example 9 below. (A) Histogram. Black lines represent the groups stained with specific anti-SSEA-4 antibody, gray lines represent the groups stained with the corresponding isotype-matched antibody. Average value ± SEM (%).
[Fig. 7] Fig. 7 shows a graph showing the positive rate in the analysis test of cell surface antigen by flow cytometry in Example 9 below. n = 3. 15% FBS: an isolation group using 15% FBS, 13% FBS: an isolation group using 13% FBS, 10% FBS: an isolation group using 10% FBS, 7% FBS: an isolation group using 7% FBS, 5% FBS: an isolation group using 5% FBS. Bar = average value ± SEM (%).***: P < 0.005.

### BEST MODES FOR CARRYING OUT THE EMBODIMENTS

The present invention relates to a method for preparing dental pulp stem cells from dental pulp cells collected from dental pulp tissue, and includes the following steps (a) to (c):
(a) preculturing the dental pulp cells using a culture medium containing serum at 7 to 13% (V/V), and attaching the dental pulp cells on a culture dish;
(b) culturing the dental pulp cells, which are attached on the culture dish in the step (a), at least for one day using a culture medium containing serum at 7 to 13% (V/V) or a serum-free culture medium for stem cells; and
(c) culturing the dental pulp cells in a low serum condition or a serum-free condition after the step (b).

In the embodiment of the present invention, "preparation" can be read as "isolation." In other words, as one embodiment, the present invention also provides a method for isolating dental pulp stem cells from dental pulp cells collected from dental pulp tissue, and the method including the steps (a) to (c).

Here, the "dental pulp tissue" in the present specification can be collected from any of the deciduous tooth and permanent tooth, and can be obtained from the dental pulp of extracted teeth such as deciduous teeth or wisdom teeth which have been conventionally discarded as medical waste. Dental pulp tissue can be removed from dentally extracted teeth at dental care facilities, and may be removed from naturally extracted or exfoliated teeth. Meanwhile, a method for removing the dental pulp tissue from teeth is known and can be appropriately implemented by those skilled in the art. In addition, when it is not possible to immediately freeze teeth on the spot, such as teeth extracted dentally, teeth should be placed in a medium such as alpha-minimum essential medium (alpha-MEM) for transportation, and it can be soaked and stored at low temperature (for example, at 4°C).

In the present specification, the "dental pulp cells collected from dental pulp tissue" is not limited to dental pulp cells collected immediately from dental pulp tissue collected from a living body, but also includes dental pulp cells collected from thawed dental pulp tissue after cryopreservation.

The origin of dental pulp tissue is not limited to humans, and may be other mammals (for example, mice, rats, rabbits, dogs, cats, monkeys, sheep, cattle, and horses).

Dental pulp cells can be obtained from dental pulp tissue, and dental pulp cells derived from the dental pulp tissue include dental pulp stem cells. Dental pulp stem cells are a type of tissue stem cells that can be isolated from dental pulp. Tissue stem cells are also called somatic stem cells, and unlike embryonic stem cells that can differentiate into all type of cells, the tissue stem cells t can differentiate into a limited type of cells.

A method for collecting dental pulp cells from dental pulp tissue is also publicly known, and can be carried out, for example, according to the method described in Example 1 below. Specifically, after shredding the dental pulp tissue using a scalpel, etc., the shredded dental pulp tissue is treated with enzymes such as dispase, collagenase, or a mixed enzyme solution thereof. The enzyme treatment can be carried out at 37°C, for 60 minutes, for example, when using a mixed enzyme solution in which 4% dispase and 3% collagenase are mixed 1:1, although it depends on the enzyme used. After the enzyme treatment, the cell suspension are thoroughly mixed with a culture medium containing serum, and then the cells are sorted and contaminants are removed using a cell strainer, etc. Thereafter, the sorted suspension is centrifuged (for example at 2,000 rpm at 4°C for 5 min). The supernatant is discarded, and dental pulp cells can be collected from dental pulp tissue by adding the culture medium. As described above, methods for collecting dental pulp cells from dental pulp tissue are publicly known, and those skilled in the art can appropriately set the reagents and test conditions, and conduct these methods.

Moreover, the "dental pulp stem cells" obtained by the method of the present invention are isolated in a state in which stem cell properties are enhanced.

Here, in the present specification, the stem cell properties of dental pulp stem cells refer to the abilities to maintain the multipotency and cell division ability without becoming differentiated. The cellular properties of such dental pulp stem cells can be evaluated by whether the expression of any one selected from the group consisting of CD146, SSEA-4, and CD34 is enhanced. In a preferred embodiment, the dental pulp stem cells obtained by the present invention have at least enhanced expression of SSEA-4 as compared to the dental pulp stem cells obtained by the conventional dental pulp stem cell isolation method (CFU-F colony formation method using culture medium containing 15% FBS). In a particularly preferred embodiment, in addition to SSEA-4, expression of CD146 or CD34 is further enhanced. Specifically, it is possible to obtain the dental pulp stem cells in which (i) the expression of CD146 and SSEA-4 or (ii) the expression of SSEA-4 and CD34 is enhanced, and in a further preferred embodiment, the dental pulp stem cells with enhanced expression of CD146, SSEA-4, and CD34 can be obtained.

Meanwhile, in the present specification, "the expression of surface antigens such as SSEA-4 is enhanced" means that the expression of specific molecules show a significant increase in the expression when the expression of these molecules are compared between the cell groups or cell colonies obtained by the method of the present invention and the cell groups or cell colonies obtained by the conventional method (CFU-F colony formation method using culture medium containing 15% FBS).

The enhanced expression of the markers of these stem cell properties can be detected using antibodies against each surface antigen, and antibodies labeled with fluorescence, etc. The detection method using antibodies can be performed using a publicly-known method, for example, a method using magnetic beads, and a method using a flow cytometer, etc. As the expression of each protein of the obtained dental pulp cells or dental pulp stem cell groups may be detected, it can be detected by other publicly-known methods. Meanwhile, a preferable evaluation method is a preferable evaluation method to detect and compare the expression in cell groups by flow cytometry using labeled antibodies to each protein as described in the following examples.

The method for isolating dental pulp stem cells according to the present invention includes the step (a) "preculturing the dental pulp cells using a culture medium containing serum, and attaching the dental pulp cells on a culture dish." In this step, the dental pulp cells collected from the dental pulp tissue, etc., can be adhered on the culture dish by the preculture.

Here, the "dental pulp cells" refer to whole dental pulp cells collected from dental pulp tissue. The cells collected from the fresh dental pulp tissue by enzyme treatment, etc., can be used as the "dental pulp cells". The cells thawed from the cryopreserved dental pulp cells after the collection from the dental pulp tissues can be also used. Alternatively, the dental pulp cells obtained from the cryopreserved dental pulp tissue by enzyme treatment, etc., can be used. Desirably, it is an embodiment using dental pulp cells obtained immediately after dental pulp tissue is treated with enzyme, etc. In other words, as a preferred embodiment of the present invention, the dental pulp cells used in the step (a) are cells that have not been subcultured. In addition, it is preferable that the dental pulp cells collected from dental pulp tissue are sorted cells by a filtration process using a 70-µm cell strainer, etc., to remove impurities from cell suspension after the dental pulp tissue treated with enzyme, etc. This step enables or facilitates sorting of the adherent cells in the subsequent culture, and can contribute to the efficient isolation of ultimate dental pulp stem cells.

As the "serum" used in the present specification, publicly known serum used for culturing mesenchymal stem cells can be used, and in a preferred embodiment, FBS is used as the serum.

Furthermore, in the present specification, when a "culture medium containing serum" is referred to, a publicly-known basal medium used for culturing mesenchymal stem cells, dental pulp cells, and dental pulp stem cells can be used as the culture medium. As such a basal medium, for example, alpha-MEM, etc., can be used, although not limited thereto. The basal medium may contain antibiotics, glutamic acid, ascorbic acid, etc., in addition to the serum as long as it does not inhibit the enhancement of the stem cell properties of the obtained dental pulp stem cells.

Moreover, when a "culture medium containing serum" is referred, the serum can be used with respect to a basal culture medium in the range of the normally used concentration. As a result of intensive studies, the present inventors have improved the number of colony formations obtained after culture by using a serum concentration of 7% to 13% (V/V) in the step (a), and have also found that an increase in the number of isolatable dental pulp stem cells or dental pulp stem cells having the enhanced stem cell properties can be obtained. Meanwhile, in the step (a), a serum concentration of 10% to 13% (V/V) is more preferable, and in particular, a concentration of about 10% (V/V) is more preferable.

In addition, the "culture dish" in the present specification is not particularly limited as long as it can be used to culture cells, and commercially available cells for cell culture, etc., can be used.

In the present specification, "to attach the dental pulp cells on a culture dish" refers to culturing dental pulp cells on a culture dish containing a culture medium by seeding and incubating the dental pulp cells collected from dental pulp tissue to adhere the dental pulp cells on the culture dish. In order to adhere the dental pulp cells to the culture dish, for example, it can be achieved by culturing the cells in a culture medium for about 3 to 48 hours. More preferably, the culture is performed for about 24 to 48 hours (around 48 hours). When the culture time is shorter, the number of adherent cells decreases, and when the culture time is longer, it is not preferable because cells other than stem cells may also adhere to the dish. However, as long as the dental pulp cells seeded on the culture dish adhere to the culture dish, the time is not limited to the above range, and a person skilled in the art can appropriately set a preferable time depending on the condition of the dental pulp cells to be used, the dish and the culture medium, etc., to be used.

The preculture of the step (a) can be performed at 37°C under the 5% CO₂ condition.

Further, in the step (a), it is preferable that the dental pulp cells collected from the dental pulp tissue are seeded as single cells on culture dishes. Thereby, single cell-derived colonies can be formed as colonies finally obtained. In other words, in a preferred embodiment of the present invention, a "step of seeding the dental pulp cells so as not to adhere to other cells on a culture dish before the step (a) is carried out," thereby the method may further include "the step of forming cell colonies derived from single cells."

A method for seeding the dental pulp cells on a culture dish cell by cell without contacting to each other is publicly known, and can be appropriately carried out by those skilled in the art. In addition, as a specific embodiment, for example, a form in which the cells are seeded at a concentration of about 0.1 × 10⁶ cells in a culture dish with a diameter of 100 mm can be included. Furthermore, those skilled in the art can carry out the preparation by appropriately adjusting the concentration depending on the dish, etc., to be used.

The method for preparing dental pulp stem cells according to the present invention includes the step (b), "a step of culturing the dental pulp cells attached on a culture dish according to the step (a) at least for 1 day using a serum-containing culture medium or serum-free culture medium for stem cells."

Here, the serum-containing culture medium used in the step (b) is preferably a culture medium containing a serum concentration in the range of 7 to 13% (V/V), and 10% to 13% (V/V); and even more preferably, the culture medium contains a serum concentration in the range of about 10% (V/V). By setting the serum concentration to 7 to 13% (V / V) in the culture medium, it is possible to improve the number of colony formations and the number of isolated cells obtained after the culture, or in addition thereto, it is possible to obtain dental pulp stem cells with the enhanced stem cell properties. Moreover, the culture medium containing the serum used for the step (b) can be substituted by discarding the culture medium used for the culture of the step (a), and adding a new culture medium.

Alternatively, in the step (b), the culture can be performed using a serum-free culture medium for stem cells. Here, the serum-free culture medium for stem cells used in the present invention is a medium capable of maintaining the proliferation and stem cell properties of stem cells. For the serum-free culture medium for stem cells, a commercially available serum-free culture medium for stem cells can be used, and in particular, a serum-free culture medium for mesenchymal stem cells is preferable. Examples of such a serum cell-free medium for stem cells include, but are not limited to, EXPREP MSC Medium (BioMimetics Sympathies), STK2 (registered trademark) serum-free culture medium for mesenchymal stem cells (DS Pharma Biomedical), Mesenchymal Stem Cell Growth Medium DXF (Promo Cell), Stemgro (registered trademark) hMSC Medium (Corning), MesenCult-SF, MesenCult-XF, MesenCult-ACF (STEMCELL Technologies), TheraPEAK MSCGM-CD Mesenchymal Stem Cell Medium, Chemically Defined (Lonza), PRIME-XV (registered trademark) MSC Expansion XSFM, PRIME-XV (registered trademark) MSC Expansion SFM (Irvine Scientific), MSC NutriStem (registered trademark) XF (Biological Industries), StemXVivo Xeno-Free Human MSC Expansion Media, StemXVivo Serum-Free Human MSC Expansion Media (R&D Systems), StemMACS MSC Expansion Media kit XF, Human (Miltenyi Biotec), MesenPRO MSC SFM, MesenPRO MSC SFM XenoFree (Gibco), FibroGRO Xeno-Free Human Fibroblast Expansion Medium (Merck), PL SOLUTION, PL SOLUTION GMP grade (PL BioScience), etc.

In addition, the culture of the dental pulp cells using a serum-containing culture medium or a serum-free culture medium for stem cells in the step (b) can be performed at least for one day (24 hours). The culture period of the step (b) is not particularly limited as long as the stem cells can be prepared, and can be, for example, for 1 day, 2 days, 3 days, 4 days, 5 days, or 6 days.

Here, the serum-containing culture medium used in the step (b) or the serum-free culture medium for stem cells can be used for the culture of the step (c) as is without replacement, and the culture can be continuously performed. In this method, it is considered that the culture medium becomes "low serum condition" and/or "low nutritional condition" about three or four days after the start of a culture in the step (b) although it depends on the serum concentration to be used, etc.

Meanwhile, the culture of the step (b) can be performed at 37°C under the 5% CO₂ condition.

In addition, the method for preparing dental pulp stem cells according to the present invention includes the step (c) "a step of culturing the dental pulp cells in a low serum condition or a serum-free condition after the step (b)." Through this step (c), it is possible to form cell colonies containing dental pulp stem cells in which expression of SSEA-4 is enhanced.

Here, the "low serum condition" refers to a state of a low concentration of serum contained in the culture medium; among the actions of serum, in particular, the culture medium has substantially no cell proliferation action on cultured cells other than dental pulp stem cells; and the culture medium contains only a serum concentration capable of maintaining or enhancing stem cell properties of the dental pulp stem cells. The culture in such a low serum state is not limited to the following form, for example, a form (although it is not limited to the following, for example, a form in which 0.1 × 10⁶ cells are continuously cultured in a culture medium at least for 3 days or more in 10 mL of a medium containing 15% (V/V) FBS) in which the culture medium used for culture is maintained for 3 days or more without changing the culture medium containing the serum, and the form culturing using the culture medium which does not contain the serum. In addition, when the cells are cultured using the serum-free culture medium for stem cells, it becomes the "serum-free condition."

In addition, the culture in the step (c) is not limited to the following as long as adherent colonies of the dental pulp stem cells can be obtained, but can be performed, for example, at least for 3 days. The culture in the step (c) can be performed at 37°C under the 5% CO₂ condition.

By performing the culture of the step (c) as described above, it is possible to form cell colonies containing dental pulp stem cells on a culture dish.

Furthermore, in one embodiment of the step (c), the step (c) can be performed in a "low serum condition or serum-free condition" and a state that can be "low nutritional condition."

Here, the "low nutritional condition" means the condition that it is possible to kill cells that are not resistant to nutrient starvation, while surviving dental pulp stem cells that are resistant to nutrient starvation may increase in proportion of dental pulp stem cells.

In the step (c), when the dental pulp cells are cultured in a "low serum condition or serum-free condition" and a "low nutrient state," the present invention is not limited to the following form, for example, a form culturing the cells with a culture medium used for cell culture for 3 days or more without replacing the culture medium (although it is not limited to the following, for example, a form in which the cells are continuously cultured in a culture medium in which 0.1 × 10⁶ cells are cultured at least for 3 days or more in 10 mL of a medium containing 15% (V/V) FBS), or a form culturing the cells using a culture medium containing no nutrient components such as serum and glucose or a culture medium containing low concentrations of nutrient components (such as physiological isotonic buffer solution). Thus, in addition to the "low serum condition," culturing the dental pulp cells in the "low nutritional condition" is preferable because it is possible to improve the proportion of the dental pulp stem cells that can be finally obtained.

In one embodiment, the present invention includes, in the step (c), culturing dental pulp cells using the culture medium containing serum used in the step (b) without replacing the medium with a new culture medium. According to such an embodiment, it is preferable that preparation and isolation of the dental pulp stem cells can be carried out simply, eliminating the need for a medium replacement operation. Moreover, the culture of the step (b) and step (c) is not limited as long as the dental pulp stem cells can be prepared, but it can be, for example, about one week (168 hours) as a standard. As a mode of an embodiment, this culture period can be set as at least 168 hours or more.

The present invention can also include, in one embodiment, a washing step for replacing the culture medium after the step (a) or step (b). The washing step is carried out for the purpose of replacing the culture medium used for the culture, and can be carried out by a publicly known method. For example, the culture medium in the culture dish can be discarded and the culture dish can be washed with sterile PBS. The washing step may be repeatedly carried out, preferably about three times.

In one embodiment, the present invention can further include at least one step of "a step of washing for replacing the culture medium (a') after the step (a) and before the step (b)," or "a step of washing for replacing the culture medium (b') after the step (b) and before the step (c)." The adherent cells can be sorted more efficiently by performing "a washing step for replacing the culture medium (a') after the step (a) and before the step (b)."

The present invention also provides, in another aspect, a method for producing a therapeutic implant material using the dental pulp stem cells prepared by the method for preparing dental pulp stem cells as described above.

The dental pulp stem cells contained in the therapeutic implant material may contain the dental pulp stem cells prepared by the dental pulp stem cell preparation method of the present invention as they are, or in order to make the dental pulp stem cells more suitable for desired therapeutic applications, it may also contain the dental pulp stem cells cultured and undergone differentiation induction under a certain condition. The method for preparing the dental pulp cells or dental pulp stem cells as grafts for therapeutic applications is not limited to the following, but for example, it can also be carried out with reference to the method for producing a therapeutic implant material for nerve damage described in WO 2014/185470.

The therapeutic implant materials may be injected into the target site by a syringe, etc., or the target site may be incised to directly place the therapeutic implant materials, and it can be used in a preferred form depending on the target disease or site. The therapeutic implant materials may also be used in combination with immunosuppressants and other medications.

Hereinafter, the present invention will be described in more detail by way of examples, but the present invention is not limited thereto. Also, the literatures cited herein are incorporated herein by reference.

### EXAMPLE

In the isolation of deciduous tooth stem cells based on the CFU-F colony formation method (Friedenstein et al., 1976), cell colonies were conventionally formed using a culture medium containing 15% FBS concentration (Yamaza et al., 2010). In the following examples, the optimum culture conditions for maintaining stem cell properties (particularly expression of surface antigens and cell proliferation ability) were examined, including changes in serum conditions in a culture for isolation of dental pulp stem cells.

### (Example 1 Dental pulp cell isolation and CFU-F colony formation method)

Three human deciduous teeth extracted from different donors were prepared. The human extracted deciduous teeth were provided by Advanced Cell Technology and Engineering Ltd. (formerly Regenerative Medicine Promotion Organization). The collected dental pulp tissue was shredded with a scalpel (Surgical Blade No. 23 manufactured by Kai Medical) in alpha-MEM. The shredded dental pulp tissue was treated with mixed enzyme solution containing 3 mL of 4% dispase (Godo Shusei) and 3 mL of 3% collagenase (Worthington Biochemicals) (both dissolved in sterile PBS) (total 6 mL) at 37°C for 60 minutes. After the enzyme treatment, 6 mL of alpha-MEM (manufactured by Life Technology) containing 15% FBS (manufactured by Equitech-Bio, SFBM30-2301) was added and thoroughly mixed, and then, the enzyme-treated solution was filtered with a 70 µm cell strainer (manufactured by BD Bisoscience) to remove the impurities. Thereafter, the mixture was centrifuged at 2,000 rpm at 4°C, and the supernatant was discarded. Then, the mixture was thoroughly mixed with 1 mL of alpha-MEM. After counting the number of cells, the cells in the obtained solution were cultured by a two-stage culture method including preculture using three kinds of culture media in which the FBS concentration was changed as described below. The concentrations of FBS in the culture media were the same between the two-step culture methods.

Nucleated cells were seeded at a concentration of 0.1 × 10⁶ per culture dish with a diameter of 100 mm (manufactured by BD Bisoscience). The culture was carried out using 10 mL of alpha-MEM culture medium containing 5%, 10% or 15% FBS, 100 mM L-ascorbic acid 2-phosphate (Wako Pure Chemical Industries), 2 mM L-glutamine (Nacalai Tesque) and 100 U/mL penicillin/100 mg/mL streptomycin mixed solution (manufactured by Nacalai Tesque), and preculture was performed at 37°C under the 5% CO₂ condition. Forty-eight hours after the start of the preculture, it was confirmed that the cells were attached on the culture dish one by one without adhesion between the cells. After confirming the cell adhesion, the culture medium was discarded and the culture dish was washed with 3 mL of sterile PBS, and the washing step was repeated three times. After the washing step, 10 mL of alpha-MEM culture medium containing 5%, 10% or 15% FBS was added. At this time, the culture medium used had the same concentration as the FBS concentration in the culture medium used for the preculture. After that, in the latter half of the culture, the cells were cultured at 37°C and under the 5% CO₂ condition for one week without any medium exchange so as to create the "low serum condition," which is the survival limit of cells (The latter half of the culture also corresponds to the "low nutritional condition.").

As a result, the formation of colonies could be confirmed under a microscope in cells attached to the culture dish by performing culture for one week. Regarding the obtained adhesion colonies, confirmation of the number of colony formations and the number of cells were attempted in the subsequent tests.

### (Example 2 CFU-F analysis)

CFU-F analysis was performed in the adherent colonies obtained by the culture of Example 1 as described below.

Specifically, after the culture of Example 1, the culture medium was discarded, the culture dish was washed with 3 mL sterile PBS, and then the washing step was repeated three times. The culture dishes were fixed with PBS buffered 4% paraformaldehyde (pH 7.4) for 15 minutes at room temperature. After the fixation, the culture dish was washed with 3 mL sterile PBS, and the washing step was repeated three times. After the washing step, staining was performed with PBS buffer 0.5% toluidine blue solution at room temperature for 2 nights. After the staining process, the culture dish was washed with distilled water and dried at room temperature. In addition, adherent colonies in the culture dish were scanned using a slide scanner to count the number of adherent colonies.

The results are shown in Fig. 1. By a culture method in which culture medium with 15%, 10% or 5% FBS concentration was used and no medium change was conducted, plastic dish-adherent colonies (cell population) composed of fibroblast-like cells, colony-forming unit-fibroblast (CFU-F) -like colonies were observed even when each concentration of FBS was used (Fig. 1A). In addition, a cell population consisting of 50 or more cells was regarded as a CFU-F colony, and CFU-F colonies formed on culture dishes were counted. The result of the colony formation number is shown in Fig. 1B. As shown in Fig. 1B, the number of formed colonies was significantly larger in the group using the culture medium with 10% FBS concentration than that in the group using the culture medium with 15% FBS concentration (p = 0.0009 vs. 15% FBS group) (Fig. 1B). On the other hand, when the group using the culture medium with 10% FBS concentration was compared with the group using the culture medium with 5% FBS concentration, no difference was found in the number of colonies.

### (Example 3 Isolated cell number analysis)

Next, the following test was performed in order to confirm the effects of two-step culture performed at each FBS concentration on the number of cells contained in CFU-F colonies formed in the culture dish.

After confirming that adherent colonies were formed by the culture in Example 1 above, the culture medium was discarded, the culture dish was washed with 3 mL sterile PBS, and the washing step was repeated three times. After the washing step, the PBS used for washing was discarded, 1 mL of a mixed solution (Nacalai Tesque) containing 2.5 g (W/V) trypsin and 1 mM EDTA was added, and enzyme treatment was performed at 37°C for 5 minutes. Then, 4 mL of alpha-MEM culture medium containing 5%, 10% or 15% FBS was added and the cells were suspended. At this time, the FBS concentration in the FBS-containing alpha-MEM culture medium was matched with the concentration used for the culture. Thereafter, the mixture was centrifuged at 2,000 rpm at 4°C. After centrifugation, the supernatant was discarded and the cells were thoroughly mixed with 1 mL of alpha-MEM. After that, the number of cells was counted. Hereinafter, each group using the alpha-MEM culture medium containing 5%, 10% or 15% FBS is referred to as 5% FBS group, 10% FBS group or 15% FBS group. The results are shown in Figs. 1C and 1D.

Fig. 1C shows the measurement results of the number of cells contained in one culture dish. When the total number of cells on the culture dish was counted, as shown in Fig. 1C, the isolated group using the culture medium with 10% FBS concentration had a significantly larger number of cells than either the group using the culture medium with 5% or 15% FBS concentration (P = 0.0004 vs. 15% FBS group; P = 0.000004 vs. 5% FBS group) (Fig. 1C).

Fig. 1D shows the total number of cells on the culture dish measured above divided by the number of colonies. As a result, in the 15% FBS group or in the 10% FBS group, approximately equivalent cell numbers were obtained (Fig. 1D). On the other hand, in the 5% FBS group, the number of cells was significantly smaller compared to the 15% FBS group or the 10% FBS group (P = 0.0006 vs. 15% FBS group; P = 0.000003 vs. 10% FBS group).

### (Example 4 Cell proliferation analysis)

After confirming that adherent colonies were formed by the culture method described in Example 1 above, the culture medium was discarded, and the culture dish was washed with 3 mL sterile PBS. The washing step was repeated three times. After the washing step, 1 mL of a mixed solution of 2.5 g (W/V) trypsin and 1 mM EDTA (Nacalai Tesque) was added, and enzyme treatment was performed at 37°C for 5 minutes. After enzyme treatment, 4 mL of alpha-MEM culture medium containing 15% FBS was added, and the cells were suspended. Then, centrifugation was carried out at 2,000 rpm and 4°C. After centrifugation, the supernatant was discarded and the cells were thoroughly mixed with 1 mL of alpha-MEM. Thereafter, the number of cells was counted. Nucleated cells were seeded at a concentration of 10 × 10³ cells/well in a 96-well plate containing 200 µL of alpha-MEM culture medium containing 15%, 10% or 5% FBS. The culture was performed at 37°C under the 5% CO₂ condition. One day after the start of the culture, Brd-U reagent (manufactured by Roche) was added. After 24 hours from the addition of the Brd-U reagent, cultured cells were treated with Cell Proliferation ELISA (manufactured by Roche), and cell proliferation was analyzed using a microplate reader.

The results are shown in Fig. 2. Fig. 2A shows the culture results of cell proliferation ability analysis in a culture medium containing 15% FBS in cell groups in which preculturing and colony formation were performed at each FBS concentration. As shown in Fig. 2A, the group precultured at 10% FBS concentration (10% FBS preculture group) showed the highest cell proliferation ability. The 10% FBS preculture group had a significantly increased cell proliferation ability (p = 0.033 vs. 15% FBS preculture group) as compared with the 15% FBS preculture group (p = 0.033 vs. 15% FBS preculture group). On the other hand, no difference was observed as compared with the 5% FBS preculture group (Fig. 2A).

Next, when culture for the cell proliferation ability analysis was performed under a 10% FBS-containing culture medium, the 10% FBS preculture group showed the highest cell proliferation ability. The 10% FBS preculture group had a significantly increased cell proliferation ability (p = 0.006 vs. 15% FBS group) compared with the 15% FBS preculture group. On the other hand, no difference was observed as compared with the 5% FBS preculture group (Fig. 2B) .

In addition, in the test where culture for the cell proliferation ability analysis was carried out with a 5% FBS-containing culture medium, the 10% FBS preculture group showed the highest cell proliferation ability, however, no significant difference was observed between the 5% FBS whole culture group or the 15% FBS whole culture group (Fig. 2C).

Furthermore, when culture for the proliferation ability analysis was performed with the same FBS concentration as that used for preculture and colony formation, the 10% FBS group showed the highest cell proliferation ability. The 10% FBS group had significantly increased cell proliferation ability (p = 0.006 vs. 15% FBS group) compared with the 15% FBS group. On the other hand, no difference was observed as compared with the 5% FBS isolated group (Fig. 2D).

### (Example 5 Flow cytometry analysis)

After confirming that adherent colonies were formed by the culture in Example 1 above, the culture medium was discarded, and the culture dish was washed with 3 mL sterile PBS. The washing step was repeated three times. After the washing step, 1 mL of a mixed solution of 2.5 g (W/V) trypsin and 1 mM EDTA (Nacalai Tesque) was added, and enzyme treatment was performed at 37°C for 5 minutes. Then, 4 mL of alpha-MEM culture medium containing 5%, 10% or 15% FBS (using the same FBS concentration as used for preculture and colony formation) was added, and the cells were suspended. Then, it was centrifuged at 2,000 rpm and 4°C. After centrifugation, the supernatant was discarded, and the cells were thoroughly mixed with 1 mL of alpha-MEM. Thereafter, the number of cells was counted. Next, nucleated cells were seeded at a concentration of 0.1 × 10⁶ in a culture dish with a diameter of 100 mm. Each isolated group was cultured at 37°C under the 5% CO₂ condition using 10 mL of a culture medium having the same FBS concentration as the culture medium used for isolation. During the culture, the culture medium was changed every three days. After 10 days from the start of the culture, the cells were detached and collected from the culture dish. Specifically, it was performed as follows. The culture medium was discarded after the culture, the culture dish was washed three times with 3 mL sterile PBS, then a mixed solution containing 1 mL of 2.5 g (W/V) trypsin and 1 mM EDTA (Nacalai Tesque)was added, and the cells were subjected to enzyme treatment at 37°C for 5 minutes. After the enzyme treatment, 4 mL of alpha-MEM culture medium containing 15% FBS was added, and the cells were suspended. Thereafter, the cells were centrifuged at 2,000 rpm and 4°C, the supernatant was discarded, and the cells were recovered by thorough mixing with 1 mL of alpha-MEM. The collected cells were dispensed as 0.1 × 10⁶ cells/100 µL PBS into FACS tubes (BD Bioscience). After dispensing, the cells were reacted with 1 µg/mL of a fluorescence-labeled antibody (Biolegend) against CD146, CD105, CD73, CD90, CD29, SSEA-4, HLA-DR, CD34, CD45, and CD14 for 45 minutes on ice for immunostaining, and then, analysis was performed with FACS Verse, a flow cytometer.

The results are shown in Fig. 3A. As shown in Fig. 3A, hematopoietic cell markers CD45 and CD14 were also negative when performing two-step culture at each FBS concentration. On the other hand, CD34 was negative in the 15% FBS group and 5% FBS group, but was significantly increased to 7.4 ± 0.3% (mean ± SEM) in the 10% FBS group (P = 0.0001 vs. 15% FBS group, 10% FBS group) (Fig. 3B). HLA-DR showed negative in each FBS concentration group (Fig. 3). In addition, markers of mesenchymal stem cells, CD146, CD105, CD73, CD90 and CD29, were positive in each FBS concentration group. CD105, CD73, CD90 and CD29 showed a positive rate of 94% or more in all groups. However, the expression of CD146 was 15.6 ± 0.1% in the 15% FBS group, 14.9 ± 0.1% in the 10% FBS group, and 9.0 ± 0.0% in the 5% FBS group, and a difference was observed between each FBS concentration group. For the expression of CD146, the expression in the 5% FBS group was reduced significantly (P = 0.000001 vs. 15% FBS group; P = 0.000002 vs. 10% FBS group) as compared with other groups (Fig. 3B).

A marker for pluripotent stem cells, SSEA-4, was also positive in each FBS concentration group. It was 35.06 ± 0.12% in the 15% FBS group, 42.23 ± 0.23% in the 10% FBS group, and 26.00 ± 0.27% in the 5% FBS concentration isolated group, there was a difference between each FBS concentration group, and the expression of SSEA-4 was significantly increased in the 10% FBS as compared with other FBS concentration groups (P = 0.000001 vs. 15% FBS group, 10% FBS group) (Fig. 3B).

As mentioned above, in the analysis of surface antigens by flow cytometry, the group isolated in 15% FBS medium satisfied the essential conditions of the immunological phenotypes of mesenchymal stem cells proposed by The International Society for Cellular Therapy (CD105, CD73, CD90: positive and CD34, CD45, CD14, HLA-DR: negative) (Dominic et al., 2006).

Therefore, the cells derived from the deciduous tooth dental pulp tissue obtained by CFU-F colony formation-based isolation method are considered to be cells belonging to mesenchymal stem cells. Similar phenotypes were observed in the 5% FBS medium isolated group as well.

On the other hand, in the 10% FBS medium isolated group, CD34 was significantly increased as compared with the other groups. The presence of CD34 positive cells has been reported in human bone marrow mesenchymal stem cells (Simmons and Torok-Storb, 1991). However, CD34 positive mouse bone marrow hematopoietic stem cells (HSCs) show CD34 positive in short-term self-renewing HSCs (ST-HSCs), but show CD34 negative in long-term self-renewing HSCs (LT-HSCs) (Spangrude et al., 1998). LT-HSCs exhibit G0 phase and very low cell proliferation rate as compared with ST-HSCs (Cheshier et al., 1999).

The existence of CD34-positive deciduous tooth stem cells has not been clarified until now, and the CD34-positive deciduous tooth stem cell population obtained in this study was identified for the first time in the world. Therefore, it is suggested that the CD34-positive deciduous tooth stem cell population may be a stem cell population different in self-replication ability or cell proliferation ability as compared with CD34-negative deciduous tooth stem cell population. Thus, it is suggested that isolation with a medium containing 10% FBS makes it possible to isolate a deciduous tooth stem cell group with enhanced expression of CD34 compared with the 15% and 5% FBS isolated groups.

In addition, in this study, the expression of CD146 was observed in isolated cell groups by any FBS concentration. Interestingly, the expression was significantly increased in the 10% FBS isolated group as compared with other concentration isolated groups. Therefore, compared to the 15% and 5% FBS isolated groups, it is suggested that the 10% FBS isolated deciduous tooth stem cell group may contain more early stem cells.

In addition, the expression of SSEA-4 was significantly increased in the 10% FBS isolated group as compared with the isolated group at other concentrations in this study. Therefore, it is suggested that by isolating the cells in a 10% FBS-containing medium, it is possible to isolate a deciduous tooth stem cell population that is primitive and has enhanced stem cell properties as compared with the conventional method (using 15% FBS).

Accordingly, in the following examples, the relationships of the concentration of serum with the cell proliferation ability and stem cell properties were further examined. Specifically, the stem cell properties (surface antigen expression and cell proliferation ability) at the serum concentration of 15%, 13%, 10%, 7%, and 5% were evaluated.

### (Example 6 Dental pulp cell isolation and CFU-F colony formation method)

Three human deciduous teeth extracted from different donors were prepared. The human extracted deciduous teeth were provided by Advanced Cell Technology and Engineering Ltd. The collected dental pulp tissue was shredded with a scalpel (Surgical Blade No. 23 manufactured by Kai Medical) in alpha-MEM. The shredded dental pulp tissue was treated with mixed enzyme solution containing 3 mL of 4% dispase (Godo Shusei, type II) and 3 mL of 3% collagenase (Worthington Biochemicals) (both dissolved in sterile PBS) (total 6 mL) at 37°C for 60 minutes. After the enzyme treatment, 6 mL of alpha-MEM (manufactured by Life Technology) containing 15% FBS (manufactured by Equitech-Bio, SFBM30-2301) was added and thoroughly mixed, and then, the enzyme-treated solution was filtered with a 70 µm cell strainer (manufactured by BD Bisoscience) to remove the impurities. Thereafter, the mixture was centrifuged at 2,000 rpm at 4°C, the supernatant was discarded. Then, the mixture was thoroughly mixed with 1 mL of alpha-MEM. After counting the number of cells, the cells in the obtained solution were cultured by the two-stage culture method including preculture using five kinds of culture media in which the FBS concentrations were changed as described below. The concentrations of FBS in the culture media were the same between the two-step culture methods.

Nucleated cells were seeded at a concentration of 0.1 × 10⁶ per culture dish with a diameter of 100 mm (BD Bisoscience). The culture was carried out using 10 mL of alpha-MEM culture medium containing 5%, 7%, 10%, 13%, or 15% FBS, 100 mM L-ascorbic acid 2-phosphate (Wako Pure Chemical Industries), 2 mM L-glutamine (Nacalai Tesque, Inc.) and 100 U/mL penicillin/100 mg/mL streptomycin mixed solution (manufactured by Nacalai Tesque), and preculture was performed at 37°C under the 5% CO₂ condition. Forty-eight hours after the start of the preculture, it was confirmed that the cells were attached on the culture dish one by one without adhesion between the cells. After confirming the cell adhesion, the culture medium was discarded. The culture dish was washed with 3 mL of sterile PBS, and the washing step was repeated three times. After the washing step, 10 mL of alpha-MEM culture medium containing 5%, 10%, or 15% FBS was added. The FBS concentration in the culture medium used for this step had the same concentration as that for the preculture. After that, the cells were cultured at 37°C and under the 5% CO₂ condition for one week without any medium exchange.

As a result, by performing culture for 1 week, formation of colonies could be confirmed under a microscope from cells adhering to the culture dish. For the obtained adhesion colonies, confirmation of the number of colony formations and the number of cells were tried in subsequent studies.

### (Example 7 CFUF-F analysis)

CFU-F analysis was performed on the adherent colonies obtained by the culture of Example 6 as described below.

Specifically, after the culture of Example 6, the culture medium was discarded, the culture dish was washed with 3 mL sterile PBS, and the washing step was repeated three times. The culture dishes were fixed with PBS buffered 4% paraformaldehyde (pH 7.4) for 15 minutes at room temperature. After the fixation, the culture dishes were washed with 3 mL sterile PBS, and the washing step was repeated three times. After the washing step, staining treatment was performed with PBS buffer 0.5% toluidine blue solution at room temperature for 2 nights. After the staining treatment, the products were washed with distilled water and were dried at room temperature. In addition, adherent colonies in the culture dish were scanned using a slide scanner to count the number of adherent colonies.

The results are shown in Fig. 4. A plastic dish-adherent cell population, CFU-F composed of fibroblast-like cells was observed at any concentration of 15%, 13%, 10%, 7% and 5% of FBS (Fig. 4A). A cell population consisting of 50 or more cells was regarded as a CFU-F colony, and CFU-F colonies formed on culture dishes were counted. In the isolated group using culture media at 10% and 7% FBS concentrations, the number of formed colonies was significantly greater compared to the isolated group at 15% and 5% FBS concentrations (Fig. 4B). On the other hand, in the 13% FBS concentration medium group, no difference was observed in the number of colonies as compared with the other medium groups (Fig. 4B). The results show that the use of 10% and 7% FBS concentration culture media can increase the number of early isolated deciduous tooth stem cells.

### (Example 8 Cell proliferation ability analysis)

After confirming that adherent colonies were formed by the culture method described in Example 6 above, the culture medium was discarded, and the culture dish was washed with 3 mL sterile PBS. After the washing step, 1 mL of a mixed solution of 2.5 g (W/V) trypsin and 1 mM EDTA (Nacalai Tesque) was added, and enzyme treatment was performed at 37°C for 5 minutes. After the enzyme treatment, 4 mL of alpha-MEM culture medium containing 15% FBS was added, and the cells were suspended. Then, centrifugation was carried out at 2,000 rpm and 4°C. After the centrifugation, the supernatant was discarded and the cells were thoroughly mixed with 1 mL of alpha-MEM. Thereafter, the number of cells was counted. Nucleated cells were seeded at a concentration of 10 × 10³ cells/well in a 96-well plate containing 200 µL of alpha-MEM culture medium containing 15% FBS or alpha-MEM culture medium having the same concentration as that used for preculture. The culture was performed at 37°C under the 5% CO₂ condition. One day after the start of the culture, Brd-U reagent (manufactured by Roche) was added. After 24 hours from the addition of the Brd-U reagent, cultured cells were treated with Cell Proliferation ELISA (manufactured by Roche), and cell proliferation was analyzed using a microplate reader.

Groups of cells precultured and colonized at each FBS concentration (15%, 13%, 10%, 7%, or 5%) were cultured for cell proliferation ability analysis in the presence of a medium containing 15% FBS, the preculture group at 13%, 10%, and 7% FBS concentration showed significantly higher cell proliferation ability as compared to the 15% and 5% FBS concentration groups (Fig. 5A). On the other hand, when the cell proliferation ability was compared in groups where the culture for cell proliferation ability analysis was conducted for one week using an FBS-containing medium having the same concentration as the FBS concentration used for the preculture and colony formation, the 13%, 10%, and 7% FBS concentration groups showed significantly higher proliferation ability as compared with the 15% FBS concentration group (Fig. 5B).

This result indicates that the isolation method using 13%, 10%, or 7% FBS concentration can give greater cell proliferation ability than the conventional isolation method using 15% concentration or 5% concentration.

### (Example 9 Flow cytometry analysis)

After confirming that adherent colonies were formed by the culture in Example 6 above, the culture medium was discarded and the culture dish was washed with 3 mL sterile PBS. The washing step was repeated three times. After the washing step, 1 mL of mixed solution (Nacalai Tesque) containing 2.5 g (W/V) trypsin and 1 mM EDTA was added, and the enzyme treatment was performed at 37°C for 5 minutes. 4 mL of alpha-MEM culture medium containing 5%, 7%, 10%, 13%, or 15% FBS (using the same FBS concentration as used for preculture and colony formation) was added and the cells were suspended. Then, centrifugation was carried out at 2,000 rpm and 4°C. After the centrifugation, the supernatant was discarded, and the cells were thoroughly mixed with 1 mL of alpha-MEM. Thereafter, the number of cells was counted. Next, nucleated cells were seeded at a concentration of 0.1 × 10⁶ in culture dishes of 100 mm in diameter. Each isolated group was cultured at 37°C under the 5% CO₂ conditions using 10 mL of a culture medium having the same FBS concentration as the culture medium used for the colony formation. During the culture, the medium was changed every three days. After 10 days from the start of the culture, the cells were detached and were collected from the culture dish. Specifically, the detachment and recovery of the cells were performed as follows. The culture medium was discarded after culture, the culture dish was washed 3 times with 3 mL sterile PBS, then 1 mL of solution (Nacalai Tesque) containing 2.5 g (W/V) trypsin and 1 mM EDTA was added, and the cells were subjected to enzyme treatment at 37 °C for 5 minutes. After the enzyme treatment, 4 mL of alpha-MEM culture medium containing 15% FBS was added, and the cells were suspended. Thereafter, centrifugation was carried out at 2,000 rpm and 4°C. The supernatant was discarded, and the cells were recovered by thoroughly mixing in 1 mL of alpha-MEM. The collected cells were dispensed into tubes for FACS analysis (BD Bioscience) so as to be a concentration of 0.1 × 10⁶ cells/100 µL PBS. After dispensing, fluorescence-labeled antibody (Biolegend) 1 µg/mL against SSEA-4 was reacted on ice for 45 minutes to perform immunostaining, and the analysis was performed using FACS Verse, a flow cytometer. Meanwhile, an antigen of SSEA-4 was used as a target surface antigen for analysis.

The results are shown in Fig. 6 and Fig. 7. Compared with the 15% FBS concentration group and the 5% FBS concentration group, the expression of SSEA-4 was significantly increased in the 13% FBS concentration group and the 10% FBS concentration group (Fig. 7). On the other hand, no significant difference was observed in the 7% FBS concentration group (Fig. 7). This result shows that, by isolating the cells in medium containing 13% and 10% FBS, it is possible to isolate a dental pulp stem cell group that is primitive and has enhanced stem cell properties as compared with the conventional CFU-F isolation method using 15% serum concentration or 5% serum concentration.

From the above results of the colony formation number, isolated cell number, cell proliferation ability and surface antigen test result by flow cytometry, it was shown that it is possible to collect a deciduous tooth stem cell group that is primitive and has enhanced stem cell properties as compared with the groups isolated using the medium compared with the isolated group using culture media with other FBS concentrations by using a medium containing serum concentration of 7% to 13%, in particular 10% concentration during the time from the isolation to the colony formation.

## Claims

1. A method for preparing dental pulp stem cells from dental pulp cells collected from dental pulp tissue, comprising the steps of:
(a) preculturing the dental pulp cells using a culture medium containing serum at 7 to 13% (V/V), and attaching the dental pulp cells on a culture dish;
(b) culturing the dental pulp cells, which are attached on the culture dish in the step (a), at least for one day using a culture medium containing serum at 7 to 13% (V/V) or a serum-free culture medium for stem cells; and
(c) culturing the dental pulp cells in a low serum condition or a serum-free condition after the step (b).

2. The method for preparing dental pulp stem cells according to Claim 1, wherein the step (c) is a step of culturing the dental pulp cells in a low serum condition or a serum-free condition and in a hypoxic condition.

3. The method for preparing dental pulp stem cells according to Claim 1 or 2, wherein the method comprises a step of seeding the dental pulp cells on a culture dish so as not to adhere to other cells before the step (a), thereby forming cell colonies derived from one cell.

4. The method for preparing dental pulp stem cells according to any one of Claims 1 to 3, wherein the serum used in the steps (a) and (b) is FBS.

5. The method for preparing dental pulp stem cells according to any one of preceding Claims 1 to 4, wherein the culture medium used in the step (a) and/or (b) is a culture medium containing serum at 10 to 13% (V/V), and the method forms cell colonies containing dental pulp stem cells with enhanced expression of SSEA-4.

6. The method for preparing dental pulp stem cells according to any one of Claims 1 to 5, further comprising a step of preparing the dental pulp cells by enzyme treatment of the dental pulp tissue before the step (a).

7. The method for preparing dental pulp stem cells according to any one of Claims 1 to 6, further comprising (a') a washing step for replacing the culture medium after the step (a) and before the step (b).

8. The method for preparing dental pulp stem cells according to any one of Claims 1 to 7, wherein the step (c) is a step of culturing dental pulp cells using the culture medium containing serum or the serum-free culture medium for stem cells used in the step (b) without replacing the medium with a new culture medium.

9. The method for preparing dental pulp stem cells according to Claim 8, wherein the total culture time of the steps (b) and (c) is at least 168 hours.

10. The method for preparing dental pulp stem cells according to any one of Claims 1 to 9, wherein the dental pulp cells seeded on the culture dish in the step (a) are contained in the culture medium at a concentration of 0.01 × 10⁶ to 0.1 × 10⁶ cells/mL.

11. The method of preparing dental pulp stem cells according to any one of Claims 1 to 10, wherein the preculture time of the step (a) is 24 to 48 hours.

12. A method for producing a therapeutic implant material, comprising the step of producing a therapeutic implant material using dental pulp stem cells prepared by the preparation method according to any one of Claims 1 to 11.

13. A dental pulp stem cell obtained by the method according to any one of Claims 1 to 11, which is derived from a single cell colony in which the expression of SSEA-4 is enhanced.

14. The dental pulp stem cells according to Claim 13, wherein the cell colony further has enhanced expression of CD146 or CD34 in addition to SSEA-4.

15. A therapeutic implant material comprising the dental pulp stem cells according to Claim 13 or 14.
